# EUROPÄISCHE PATENTSCHRIFT

(11) **EP 2 654 686 B1**
(45) Veröffentlichungstag und Bekanntmachung des Hinweises auf die Patenterteilung: **16.01.2019**
(21) Anmeldenummer: 11748636.5
(22) Anmeldetag: 19.08.2011
(51) Int. Cl.: A61K 8/35, A61Q 13/00, A61K 31/045, A61K 31/11, A61K 31/121, A61K 31/365, A61K 31/366

(54) **KOSMETISCHE ZUBEREITUNGEN MIT BERUHIGENDER WIRKUNG**
COSMETIC PREPARATIONS HAVING A CALMING EFFECT
PRÉPARATIONS COSMÉTIQUES À ACTION RELAXANTE

(30) Priorität: 02.09.2010 DE 102010036179
(43) Veröffentlichungstag der Anmeldung: 30.10.2013
(60) Teilanmeldung: 18209391.4
(73) Patentinhaber: Beiersdorf AG, 20253 Hamburg (DE)
(72) Erfinder: MARTIN, Annette, 90429 Nürnberg (DE); DINGLER, Christian, 22527 Hamburg (DE); LORENZ, Janina, 22607 Hamburg (DE)
(74) Vertreter: Hartmann, Jost
(86) Internationale Anmeldenummer: PCT/EP2011/064264
(87) Internationale Veröffentlichungsnummer: WO 2012/028473

(56) Entgegenhaltungen:
- WO-A1-02/49600
- US-A1- 2004 097 598
- US-A1- 2007 231 278
- DATABASE GNPD [Online] MINTEL; September 2009 (2009-09), "Scented Candle", XP002706295, Database accession no. 1175836
- "rose oil (rosa damascena) bulgaria", The Good Scents Company , XP002706296, Gefunden im Internet: URL:http://www.thegoodscentscompany.com/gc a/gc1024951.html [gefunden am 2013-07-25]

## Beschreibung

Die Erfindung betrifft die kosmetische nichttherapeutische Verwendung des Stoffes 2,6-Dimethyl-7-octen-2-ol zur psychologischen Beruhigung und/ oder Entspannung des erwachsenen Menschen nach Anspruch 1.

Im heutigen Alltag bleibt aufgrund der hohen Arbeitsbelastung immer weniger Zeit für Erholung. Stress hat einen negativen Einfluss auf Körper und Psyche des Menschen und wirkt sich nicht zuletzt auch auf das Erscheinungsbild von Haut und Haar aus. Der Einsatz beruhigender Kosmetikprodukte, die ihre Anti-Stress-Wirkung bei topischer Applikation entfalten, kann einen wesentlichen Beitrag zur Steigerung des Wohlbefindens von Konsumenten leisten.
Viele pflanzliche ätherische Öle wie Lavendelöl, Orangenöl oder Rosenöl besitzen bekanntermaßen entspannende, angstlösenden oder stimmungsaufhellende Effekte, wie beispielsweise in Lehrner, J., Eckersberger, C., Walla, P., Potsch, G. and Deecke, L. Ambient odor of orange in a dental office reduces anxiety and improves mood in female patients. Physiol Behav. 71(1-2):83-6 (2000); in Lehrner, J., Marwinski, G., Lehr, S., Johren, P. and Deecke, L. Ambient odors of orange and lavender reduce anxiety and improve mood in a dental office. Physiol Behav. 86(1-2):92-5 (2005) oder in Hongratanaworakit, T. Relaxing effect of rose oil on humans. Nat Prod Commun. 4(2):291-6 (2009), beschrieben.
Diese Duftstoffe werden häufig im Rahmen der sogenannten Aromatherapie eingesetzt. Düfte werden beim Atmen über die Nase aufgenommen, die ihre Impulse direkt zum Gehirn weiter leitet. Einer der Informationswege aktiviert das limbische System, den Sitz der Emotionen. Durch Düfte können daher beim Einatmen Emotionen erzeugt werden.
In der EP 1415644 werden Parfumzusammensetzungen beschrieben, die zur Verhinderung oder Glättung von Ausdrucksfalten oder zur Muskelentspannung verwendet werden können. Hierin beschriebene Parfuminhaltsstoffe umfassen eine Vielzahl von Einzelsubstanzen und ein bestimmtes Verhältnis zueinander um diesen Muskelentspannungseffekt zu bewirken.
Bedingt durch das autonome Nervensystem (ANS) spiegeln sich psychologische Reaktionen daher häufig auch in einer physiologischen Reaktion wieder.

Beispielsweise führen psychische Erregungszustände, vermittelt durch das sympathische Nervensystem, zu einem Anstieg der Herzschlagrate, erhöhter Schweißsekretion oder einer Kontraktion von peripheren Blutgefäßen oder Skelettmuskelgefäßen. Dagegen sind Herzschlagverlangsamung oder Dilatation von Skelettmuskelgefäßen Indikatoren von Entspannungsreaktionen, die durch das parasympathische Nervensystem vermittelt werden (Gramann und Schandry, 2009). Durch Messung psychophysiologischer Reaktionen können Duft-vermittelte Entspannungseffekte objektiv gemessen werden. So konnte beispielsweise im Probandenexperiment durch Messung der Herzratenvariabilität und des Blutdrucks die entspannende Wirkung von Cedrol gezeigt werden, wie in Dayawansa, S., Umeno, K., Takakura, H., Hori, E., Tabuchi, E., Nagashima, Y., et al. Autonomic responses during inhalation of natural fragrance of Cedrol in humans. Auton Neurosci. 108(1-2):79-86 (2003), dargestellt. Auch für R-(-)-Linalool wurde durch Messung der Herzrate nachgewiesen, dass Probanden beim Einatmen dieses Parfumstoffs entspannen (Hoferl, M., Krist, S. and Buchbauer, G. Chirality influences the effects of linalool on physiological parameters of stress. Planta Med. 72(13(2006)).
Weitere Parameter, die für die Messung psychophysiologischer Reaktionen angewandt werden, sind dermale Aktivität, Blutvolumenpuls, Elektromyographie oder Hirnaktivität (EEG).

Objektive psychophysiologische Messungen von Dufteffekten werden idealerweise ergänzt durch psychologische Fragebögen, in denen Stimmungszustände abgefragt werden, die eine subjektive Einschätzung des Probanden wieder geben. So kann ein allumfassendes Bild der Duftwirkung erzeugt werden. Als Beispiele für psychologische Fragebögen seien hier "Profile of Mood States" (POMS), "State Trait Anxiety Inventory" (STAI), der Multi-dimensionale Befindlichkeitsfragebogen (MDBF) oder das Self Assessment Manikin (SAM) genannt. Während die drei erst genannten verbale Fragebögen sind, handelt es sich bei letzterem um einen Bild-gebundenen Fragebogen, bei dem Emotionen in den drei Dimensionen Stimmung, Dominanz und Erregung abgefragt werden (Bradly and Lang, 1994).

Es ist seit langem bekannt, dass Riechstoff-Materialien und ätherische Öle die Gefühle der Entspannung bzw. Relaxation und des Wohlbefindens fördern können, wie in der WO 2006097759 A1 und WO 0249600 A1 beschrieben.

So wird in der WO 0249600 A1 ein Verfahren zur Abgabe von positiven Gemütseinflüssen, insbesondere Relaxationseinflüssen, an eine Person, das Verabreichen einer wirksamen Menge einer Parfümzusammensetzung beansprucht.

Die als beruhigend wirkende Parfumzusammensetzung umfasst dabei eine Reihe verschiedenster Parfuminhaltsstoffe und bestimmte Verhältnisse der Stoffe zueinander.
Die Parfümzusammensetzungen zielen darauf ab positive Niedrig-Aktivierungs-Stimmungen und -Emotionen zu induzieren oder damit assoziiert zu werden bzw. auf ein Verfahren zur Abgabe von positiven Gemütseinflüssen, insbesondere Relaxation bzw. Entspannung, an ein Subjekt, insbesondere ein menschliches Subjekt, das die Abgabe der Parfümzusammensetzung in einer Form umfasst, die aus Verbraucherprodukten, die zur Aufbringung auf Haut, Haar, harte Oberflächen oder Stoffe bestimmt ist, und aus Luftpflegeprodukten, zum Beispiel Airfreshener, ausgewählt ist.

Die EP 1424071 A2 offenbart Terpinylbutyrate zum Bewirken einer mentalen Sedierung.

US 20100129312 A1 beschreibt verschiedene Duftstoffe, die auf Neugeborene und Babys anziehend wirken. Nahrung und Gegenstände, wie Spielzeug, oder auch Betreuungspersonen, die mit diesen Duftstoffen versehen sind, sollen somit die Akzeptanz der Babys erhöhen.
Aus wissenschaftlichen Studien ist weiterhin bekannt, dass sich die Geruchsrezeptoren ändern können und Kinder bis zur Pubertät ein den Erwachsenen unterschiedliches Wahrnehmungsvermögen für Duftstoffe aufweisen.
(Koelega HS: Prepubescent children may have specific deficits in olfactory sensitivity; Percept Mot Skills; 1994; Feb; 78(1):191-9;
Doty RL, Shaman P, Applebaum SL, Giberson R, Siksorski L, Rosenberg L.: Smell identification ability: changes with age; Science 1984; Dec 21; 226(4681):1441-3)

Im heutigen Alltag bleibt aufgrund der hohen Arbeitsbelastung immer weniger Zeit für Erholung. Stress hat einen negativen Einfluss auf Körper und Psyche des Menschen und wirkt sich nicht zuletzt auch auf das Erscheinungsbild von Haut und Haar aus.
Der Einsatz beruhigender Kosmetikprodukte, die Ihre Anti-Stress-Wirkung bei topischer Applikation entfalten, kann einen wesentlichen Beitrag zur Steigerung des Wohlbefindens von Konsumenten leisten.
Wünschenswert ist es daher, Kosmetikprodukte mit beruhigenden Wirkeffekten zu entwickeln.

Wünschenswert ist es darüber hinaus konkrete Einzelsubstanzen zur Verfügung zu stellen, die eine beruhigende oder entspannende Wirkung im psychologischem Sinne auf den Menschen, insbesondere dem erwachsenen Menschen ausüben. Wünschenswert ist es darüber hinaus, dass diese Substanzen in kosmetischen Zubereitungen zur Anwendung kommen können.

In diesem Sinne ist es erstrebenswert, Parfumsubstanzen einzusetzen, die gut verträglich sind und nicht über allergene Eigenschaften verfügen, da beim Konsumenten durch Duftstoffallergien negative Emotionen ausgelöst werden könnten, die einer entspannenden Wirkung entgegen stehen. Zu den in diesem Sinne kritischen Parfumsubstanzen gehören gemäß einer Studie des SCCP Amylcinnamal, Benzylalkohol, Cinnamylalkohol, Citral, Eugenol, Hydroxycitronellal, Isoeugenol, Amylcinnamylalkohol, Benzylsalicylat, Cinnamal, Cumarin, Geraniol, Hydroxy-Methylpentyl-cyclohexencarboxaldehyd, Anisalkohol, Benzylcinnamat, Farnesol, 2-(4-tert-Butylbenzyl)propioaldehyd, Linalool, Benzylbenzoat, Citronellol, Hexylcinnamaldehyd, d-Limonen, Methylheptincarbonat, 3-Methyl-4-(2,6,6-trimethyl-2-cyclohexen-1-yl)-3-buten-2-on und Eichenmoos- sowie Baummoosextrakt.
Ziel war es daher, beruhigende Duftstoffe zu identifizieren, die nicht zu den allergen genannten Verbindungen zählen.

Überraschenderweise wurden 6-Methyl-5-Hepten-2-one, Octan-2-one, Heptan-2-one, Nonan-2-one, 5-hexyldihydro-5-methyl-2(3H)-furanone, Tetrahydro-6-pentyl-2H-pyran-2-one, Octanal und 2,6-Dimethyl-7-octen-2-ol als auf den erwachsenen Menschen im obigen Sinne psychophysiologisch beruhigend bzw. entspannend wirkende Substanzen ermittelt.

6-Methyl-5-Hepten-2-one (C₈H₁₄O) mit der CAS-Nr.: 110-93-0, auch als Methylheptenone oder Methylisohexenylketon bezeichnet, der Struktur 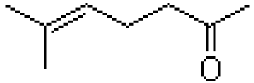 findet sich als Duftstoff vornehmlich in Früchten und Gemüse, wie Tomaten.

Octan-2-one (C8H16O) mit der CAS-Nr.: 111-13-7 der Struktur 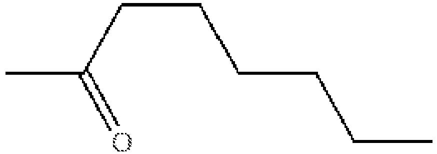 ist als Parfumrohstoffstoff bekannt.

Heptan-2-one (C₇H₁₄O) mit der CAS-Nr.: 110-43-0, auch als 2-Heptanon, n-Amylmethylketon, Methylpentylketon bezeichnet, der Struktur 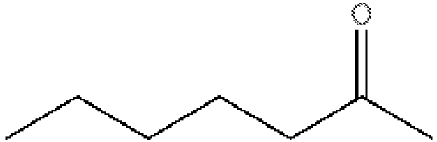 tritt mit einem fruchtigen Geruch beispielsweise in Bananen auf.
Nonan-2-one mit der CAS-Nr.: 821-55-6 und der Struktur 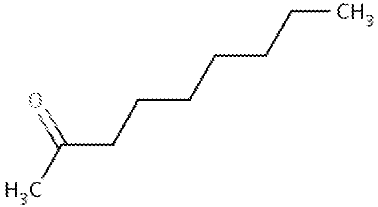 ist als Duftstoff und Aromazusatz bekannt.

5-hexyldihydro-5-methyl-2(3H)-furanone mit der CAS-Nr.: 7011-83-8, auch als Dihydrojasmone lactone bezeichnet, und der Struktur 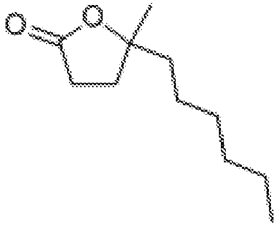 ist als Aroma im Bier zu finden (Spence et al., Technical Quarterly, Master Brewers Ass. of America 10, 127 (1973).

Tetrahydro-6-pentyl-2H-pyran-2-one mit der CAS-Nr.: 705-86-2, auch als 5-Decanolide oder delta Decanolacton bezeichnet, mit der Struktur 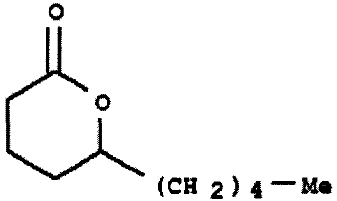 ist als Duftstoff mit einem kokosartigem Aroma bekannt.
Octanal mit CAS -Nr.: 124-13-0, auch als Caprylaldehyd oder Octylaldehyd bezeichnet, mit der Struktur 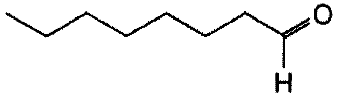 ist als Ausgangsstoff für synthetische Duftstoffe wie Rosenöl oder Citronenöl bekannt. 2,6-Dimethyl-7-octen-2-ol mit der CAS-Nr.: 18479-58-8, auch als Dihydromyrcenol bezeichnet, der Struktur 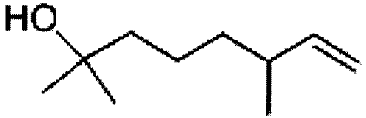 ist als Geruchsstoff mit einem Zitrusaroma bekannt.
Überraschend ist, dass die Stoffe eine beruhigende Wirkung ausüben, obwohl sie unterschiedliche Dufteindrücke, wie erdig, kokosartig, blumig oder fruchtig (zitrusartig) vermitteln.
Vorteilhaft ist dabei auch, dass die Stoffe nicht auf der o.g. SCCP-Liste der allergenen Parfuminhaltsstoffe stehen.
Die Erfindung betrifft daher die kosmetische nichttherapeutische Verwendung des Stoffes 2,6-Dimethyl-7-octen-2-ol zur psychologischen Beruhigung und/oder Entspannung des erwachsenen Menschen nach Anspruch 1.

Die Düfte werden beim Atmen über die Nase aufgenommen, die ihre Impulse direkt zum Gehirn weiter leitet. Einer der Informationswege aktiviert das limbische System. Durch Düfte können daher Emotionen erzeugt werden, denen sich der "Riechende" nicht entziehen kann.
Durch psychophysiologische Messungen konnte völlig überraschend gezeigt werden, dass die eigentlich nur als Parfumstoffe bekannten 6-Methyl-5-Hepten-2-one, Octan-2-one, Heptan-2-one, Nonan-2-one, 5-hexyldihydro-5-methyl-2(3H)-furanone, Tetrahydro-6-pentyl-2H-pyran-2-one, Octanal und 2,6-Dimethyl-7-octen-2-ol einen beruhigenden Effekt auf den erwachsenen Menschen ausüben.
In verschiedenen Test wurden verschiedenste Stoffe hinsichtlich ihrer Beruhigungswirkung untersucht. Beispielsweise wurden zur Vorauswahl der Substanzen 10 Probandinnen im Alter von 20 bis 50 Jahren gebeten, 16 Einzelduftstoffe, die 0,5 %ig in Propylenglykol verdünnt waren, zu riechen und diejenigen Duftstoffe auszuwählen, deren Duftcharakter sie persönlich als beruhigend empfanden. Die Mehrzahl der Probandinnen wählte in diesem Test Linalool und Cedrol als beruhigende Duftstoffe aus. Interessanterweise handelt es sich bei diesen beiden Substanzen um Terpene, für die bereits eine beruhigende Wirkung vorbeschrieben ist (siehe zum Beispiel obige Literaturangaben bzw. JP2009051835 A, EP1170005 A1). Zudem ist Linalool als ein typisch beruhigender Duftstoff auf der Liste der allergenen Substanzen zu finden.
Zusätzlich wurden von den Probandinnen die Riechstoffe 6-Methyl-5-Hepten-2-one, Octan-2-one, Heptan-2-one, Nonan-2-one, 5-hexyldihydro-5-methyl-2(3H)-furanone, Tetrahydro-6-pentyl-2H-pyran-2-one, Octanal und 2,6-Dimethyl-7-octen-2-ol als Düfte mit beruhigendem Duftcharakter ausgewählt. Der Duftcharakter von Octan-2-one, Heptan-2-one, Nonan-2-one kann als erdigkrautig beschrieben werden. 5-hexyldihydro-5-methyl-2(3H)-furanone, 2,6-Dimethyl-7-octen-2-ol und 6-Methyl-5-Hepten-2-one verfügen über einen eher blumig-grünen Charakter. Tetrahydro-6-pentyl-2H-pyran-2-one riecht dagegen süß-cremig und kokosartig und Octanal besitzt ein zitrusartiges frisches Duftprofil. D.h. anhand ihrer bekannten Parfumduftnote war eine für den Menschen beruhigende Wirkung dieser Stoffe nicht naheliegend. Vielmehr ist bekannt, dass Duftstoffe mit den beschriebenen frisch-grünen Dufteigenschaften eher mit einer vitalisierenden Wirkung assoziiert werden.
Entsprechend eher stimulierend wurden von den Probandinnen auch die Düfte Limonen, Citronellol und Amyl salicylate empfunden. Als neutrale Düfte wurden außerdem folgende Riechstoffe definiert: Hexyl salicylate, Phenylethyl alcohol, Methyl dihydrojasmonate, Benzyl salicylate, Benzyl benzoate, Methyl cedryl ketone.
Umso erstaunlicher ist es daher, dass die Stoffe eine beruhigende Wirkung zeigen.

Es wurde in weiteren Tests die beruhigende Wirkung der Duftstoffe 6-Methyl-5-Hepten-2-one, Octan-2-one, Heptan-2-one, Nonan-2-one, 5-hexyldihydro-5-methyl-2(3H)-furanone, Tetrahydro-6-pentyl-2H-pyran-2-one, Octanal und 2,6-Dimethyl-7-octen-2-ol auf den Menschen untersucht. Dazu wurde ein Testdesign gewählt, bei dem mit Hilfe eines Olfaktometers (Burghart) kosmetisch einsetzbare und kommerziell erhältliche Duftstoffe an die Nase des Probanden gebracht wurden. Die Duftstoffe wurden in Propylenglykol (Caelo) gelöst. Als Placebokontrolle wurde entsprechend ebenfalls Propylenglykol eingesetzt

Die Probandinnen wurden in einer bequemen, leicht nach hinten gelehnten Haltung auf einem Sessel platziert. Das Ende des Olfaktometerschlauches wurde im Abstand von etwa 10 cm vor der Nase des Probanden platziert und mit Hilfe eines Stativs fixiert. Der Versuchsaufbau (Nexus, Olfaktometer) wurde von den Probandinnen mit Hilfe einer spanischen Wand abgeschirmt, um die Teilnehmerinnen nicht durch das Messgeschehen abzulenken. Die Messungen fanden in einem hellen, gut gelüfteten, auf 21°C temperierten Raum statt.

An den Untersuchungen nahmen Probandinnen im Alter von 20 bis 40 Jahren teil. Das Durchschnittsalter betrug 31 Jahre. Die Probandinnen besaßen einen BMI zwischen 19 und 25. Die Probandinnen wurden gebeten, einen Tag vor Studienbeginn auf Parfum und Body Lotion zu verzichten. Am Messtag selbst durften die Teilnehmerinnen weder Parfum, noch parfümierte Deoprodukte, Body Lotions, Gesichtscreme, Handcreme und Haarspray benutzen. Sie mussten auf Kaffee, Schwarztee, Fruchtsäfte und Zigaretten verzichten und durften 30 min vor Testbeginn nichts mehr essen und ausschließlich Wasser trinken.

Die Einstellungen am Olfaktometer sahen einen Luftstrom von 4 L/min vor. Die externe Druckluft betrug 2 bar. Die Probandinnen wurden mit den entsprechenden Messelektroden bestückt und akklimatisierten zunächst für 1 min. Diese Zeit wurde gleichzeitig genutzt, um sicherzustellen, dass sich die Elektroden zur Bestimmung psychophysiologischer Parameter in einem schwankungsfreien und messbereiten Zustand befinden. Alle Probandinnen erhielten im Anschluss für 5 min Kontrollluft ohne Duft. Während dieser Zeit sollten alle Probandinnen einen emotional neutralen Zustand erreichen. Anschließend erhielten die Probandinnen für 5 min einen Duftstoff, der so niedrig konzentriert war, dass er nicht unangenehm oder gar stechend empfunden wurde. Alle Duftstoffe wurden in Propylenglykol gelöst. Als Placebokontrolle diente ebenfalls Propylenglykol. Während der gesamten 10 min wurden die psychophysiologischen Messparameter dermale Aktivität, Herzrate und Blutvolumenpuls mit Hilfe des Messgeräts NeXus und der Software Biotrace (Mind Media B.V.) erfasst. Die Differenz zwischen maximalem und minimalem Blutvolumen innerhalb eines Herzzyklus nennt man Blutvolumenpuls Amplitude. Dieser Wert sinkt bei psychischer Belastung ab. Umgekehrt kann ein Anstieg als Entspannungsreaktion interpretiert werden.

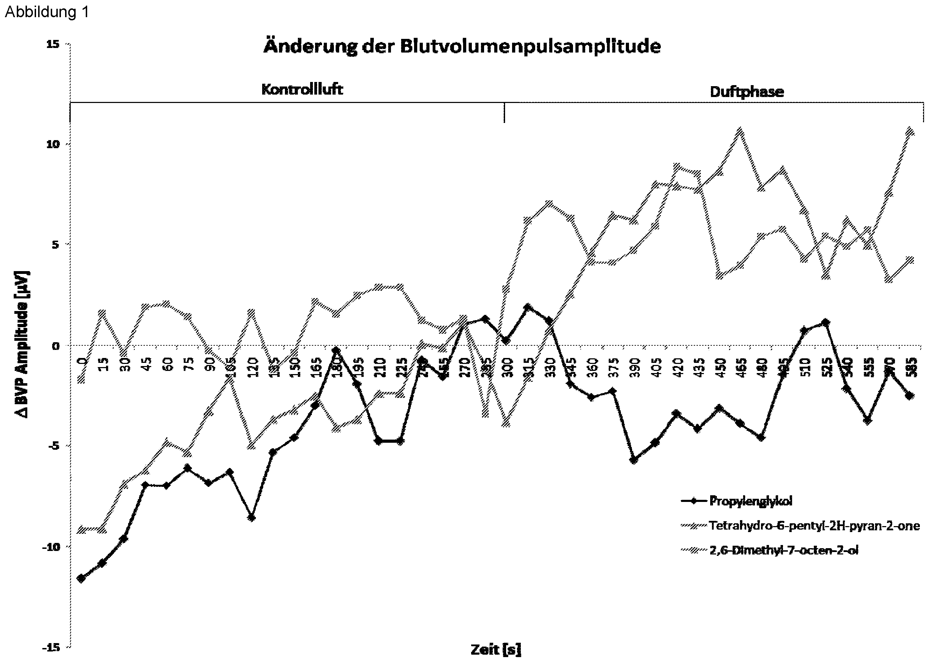

In weiteren Versuchen konnte mit dem oben beschriebenen Versuchsaufbau gezeigt werden, dass die Einzelriechstoffe Tetrahydro-6-pentyl-2H-pyran-2-one und 2,6-Dimethyl-7-octen-2-ol gegenüber der Placebokontrolle bei Probanden zu einem Anstieg der Blutvolumenpuls Amplitude führen.
Dies ist eine Duft-vermittelte Beruhigungs- bzw. Entspannungsreaktion, die auch bei 6-Methyl-5-Hepten-2-one, Octan-2-one, Heptan-2-one, Nonan-2-one und 5-hexyldihydro-5-methyl-2(3H)-furanone und Octanal beobachtet wurde. Gleichzeitig sank bei den erfindungsgemäß beruhigenden Düften die Herzrate signifikant ab. Beispielhaft sind in Abbildung 1 und Tabelle 1 die Messwerte der Untersuchungen für 0,075% 2,6-Dimethyl-7-octen-2-ol und 0,025% Tetrahydro-6-pentyl-2H-pyran-2-one dargestellt.

Abbildung 1 zeigt, dass die Einzelriechstoffe Tetrahydro-6-pentyl-2H-pyran-2-one (N=9) und 2,6-Dimethyl-7-octen-2-ol (N=10) gegenüber der Placebokontrolle, Propylenglycol (N=10) bei Probanden zu einem Anstieg der Blutvolumenpuls Amplitude führen und damit eine beruhigende und entspannende Wirkung im psychologischen Sinne haben und als solche zu kosmetischen Zwecken zu verwenden sind.

Um eine gute Vergleichbarkeit der Messdaten der verschiedenen Testgruppen gewährleisten zu können, wurden die Rohdaten der Messung normiert. Dafür wurde der Mittelwert der BVP Amplitude 1 min vor Duftapplikation von allen anderen Messwerten abgezogen. Die Summe der Differenzen ergibt die Änderung der BVP Amplitude in µV.

Während der Messung der BVP Amplitude wurde zu einigen Zeitpunkten die Herzrate mitbestimmt (Tab. 1).

**Tab. 1: Tabellarische Darstellung der Änderung der Herzrate nach Applikation des Einzelriechstoffes Tetrahydro-6-pentyl-2H-pyran-2-one im Vergleich zur Placebokontrolle.**

| Änderung der Herzrate [bpm] | | | | |
|---|---|---|---|---|
| | **Zeit[s]** | **Propylenglykol** | **Tetrahydro-6-pentyl- 2H-pyran-2-one** | **p-Wert** |
| **Kontrollluft** | **30** | **-0,04** | **-1,26** | **0,967** |
| | **90** | **0,06** | **-1,26** | **0,438** |
| | **135** | **0,59** | **-1,65** | **0,438** |
| | **180** | **-0,18** | **-2,16** | **0,775** |
| | **225** | **0,30** | **-0,63** | **0,653** |
| | **285** | **0,23** | **-0,02** | **0,838** |
| **Duflphase** | **300** | **0,81** | **-2,51** | **0,031** |
| | **315** | **1,21** | **-3,27** | **0,004** |
| | **330** | **1,16** | **-2,71** | **0,005** |
| | **435** | **0,41** | **-1,94** | **0,046** |
| | **540** | **0,20** | **-2,91** | **0,031** |
| | **585** | **0,75** | **-2,44** | **0,012** |

Im Vergleich zur Placebokontrolle ändert sich die Herzrate der Gruppe, die im Anschluss den Duft bekommt, nicht (p>0,05). Erst nach Applikation von Tetrahydro-6-pentyl-2H-pyran-2-one ändern sich im Vergleich zur Placebokontrolle die gemessenen Herzraten signifikant (p<0,05). Deutlich wird, dass der Einzelriechstoff seine beruhigende Wirkung über eine Senkung der Herzrate entfaltet. Die hier dargestellten Messwerte beschreiben die Änderung der Herzrate nach Normierung (siehe BVP Amplitude).

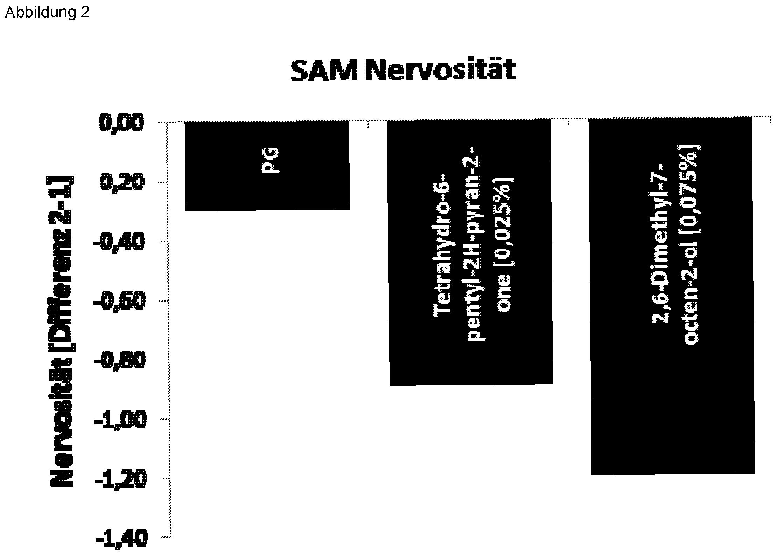

Zur Ergänzung der zuvor ermittelten Daten wurde mit den Probandinnen ein kurzer Stresstest durchgeführt, bei dem sie nach einer Anfangsentspannung für 1 min einer schnellen Folge von unangenehmen Bildern (Spinnen, Feuer, etc.) auf einem Fernsehbildschirm ausgesetzt wurden. Die visuellen Reize wurden von einem lauten Trommelrhythmus begleitet. Vor und nach dem Stresstest wurden die Probandinnen gebeten, mit Hilfe eines Self-Assessment Manikins (SAM), ihre eigene Nervosität zu bewerten.
Auch hierbei reagierten Probandinnen, die mit 6-Methyl-5-Hepten-2-one, Octan-2-one, Heptan-2-one, Nonan-2-one, 5-hexyldihydro-5-methyl-2(3H)-furanone, tetrahydro-6-pentyl-2H-pyran-2-one, Octanal oder 2,6-Dimethyl-7-octen-2-ol behandelt worden waren, im Mittel mit einer geringeren Stress-vermittelten Änderung der Nervosität als Placebo-behandelte Probandinnen. Beispielhaft sind in Abbildung 2 die Werte des SAM-Fragebogens für Nervosität für 2,6-Dimethyl-7-octen-2-ol und Tetrahydro-6-pentyl-2H-pyran-2-one dargestellt. Abbildung 2 zeigt die Angaben der Probandinnen zu ihrer Nervosität vor und nach dem Stresstest als Differenz (nachher minus vorher).

Die Nervosität nahm in allen der hier dargestellten Gruppen nach dem Stresstest ab. Im Vergleich zur Placebokontrolle (PG) sank die Nervosität bei den Duft-behandelten Probandinnen stärker, was für einen Beruhigungseffekt im psychologischen Sinne spricht.

Zusammenfassend haben alle durchgeführten Untersuchungen ergeben, dass die Duftstoffe 6-Methyl-5-Hepten-2-one, Octan-2-one, Heptan-2-one, Nonan-2-one, 5-hexyldihydro-5-methyl-2(3H)-furanone, Tetrahydro-6-pentyl-2H-pyran-2-one, Octanal und 2,6-Dimethyl-7-octen-2-ol eine beruhigende Wirkung besitzen.
Bislang ist für keinen dieser Duftstoffe ein beruhigender Effekt im psychologischen Sinne für erwachsene Menschen nachgewiesen worden. Beispielsweise ist Dihydromyrcenol zwar als möglicher Inhaltsstoff beruhigender Parfumzusammensetzungen beschrieben, wird dort aber in Hinblick auf seine emotionalen Wirkeffekte als "neutraler" Parfuminhaltsstoff und keineswegs als beruhigender Duftstoffstoff eingestuft (EP 1343466 B1, US 20070207220 A1). Bei 6-Methyl-5-Hepten-2-one handelt es sich um einen pflanzlichen Inhaltsstoff, der in geringen Konzentrationen unter anderem in Zitronenmelisse (Melissa officinalis) vorkommt. Dem ätherischen Öl der Melisse wird im Allgemeinen eine beruhigende Wirkung nachgesagt, die aber der Hauptkomponente Citral zugesprochen wird. Citral ist im ätherischen Öl der Melisse mit 40 - 70 % enthalten und überdeckt daher alle anderen ggf. vorkommenden Düfte.
Das 6-Methyl-5-Hepten-2-one als beruhigend und entspannend wirkt und entsprechend in kosmetischen Zubereitungen verwendet werden kann lag somit nicht nahe.
US 20100129312 A1 beschreibt zwar neben verschiedenen anderen Duftstoffen die akzeptanzerhöhende Wirkung von Octanal und Decalactone Delta auf Babys, jedoch ist bekannt, dass Geruchswahrnehmung und psychologische Wirkung von Duftstoffen bei Erwachsenen nicht vergleichbar ist mit der von Babys.
Erfindungsgemäß ist daher das Verfahren bzw. die Verwendung der Duftstoffe zum Bewirken einer psychologischen Beruhigung und/oder Entspannung auf erwachsene Menschen beschränkt. Bevorzugt wird daraufhin 2,6-Dimethyl-7-octen-2-ol.

Als erwachsene Menschen sind alle Menschen älter als 18 Jahre anzusehen. Nach diesem Zeitraum ist die Geruchswahrnehmung, die sich in den Pubertätsjahren verändert, nahezu entwickelt.

Um den gewünschten Beruhigungseffekt zu erzielen, ist es vorteilhaft, dass die Duftstoffe 6-Methyl-5-Hepten-2-one, Octan-2-one, Heptan-2-one, Nonan-2-one, 5-hexyldihydro-5-methyl-2(3H)-furanone, Tetrahydro-6-pentyl-2H-pyran-2-one, Octanal und 2,6-Dimethyl-7-octen-2-ol oder Mischungen dieser Stoffe die Nase erreichen, um dort über Geruchsrezeptoren wahrgenommen zu werden.
Da die genannten Stoffe bei 25 °C einen Dampfdruck unterhalb des atmosphärischen Drucks besitzen, liegen sie bei Raumtemperatur flüssig vor. Ideal ist daher eine topische Auftragung der Substanzen auf die Haut oder Haar, da durch die Temperatur der Haut von etwa 32°C ein Übergang der Stoffe in die Gasphase gewährleistet ist. Dies kann in Form eines Eau de Toilettes, eines Eau de Parfums, eines Bodysprays oder eines Haarsprays erfolgen, das auf die Haut oder das Haar aufgebracht wird. Ideal ist darüber hinaus die Applikation der Einzeldüfte oder Mischungen der Duftstoffe in kosmetischen Zubereitungen vorteilhaft in Form von wässrigen Gelen, wässrig-alkoholischen Lösungen, O/W- oder W/O-Emulsionen von weicher, halb-weicher oder fester Konsistenz, Mikroemulsionen, W/O/W- oder O/W/O-Emulsionen. Ideale Applikationsformen sind in diesem Zusammenhang Gesichtscremes, Seren, Make-up, Foundation, Body Lotions, Body Milks, Handcreme, Deo-Roller, Deo-Stick, Deo-Aerosol, Deo-Zerstäuber. Auch der Einsatz in rinse-off-Produkten wie Shampoos, Duschgelen oder Handwaschgelen ist vorteilhaft, da durch Kontakt mit warmem Wasser die o.g. beruhigenden Duftstoffe ebenfalls in die Gasphase übergehen.

Neben den genannten kosmetischen Einsatzmöglichkeiten, können die Duftstoffe 6-Methyl-5-Hepten-2-one, Octan-2-one, Heptan-2-one, Nonan-2-one, 5-hexyldihydro-5-methyl-2(3H)-furanone, Tetrahydro-6-pentyl-2H-pyran-2-one, Octanal und 2,6-Dimethyl-7-octen-2-ol auch in Form von Duftkerzen, Öllämpchen oder Raumbeduftungssystemen eingesetzt werden um einen psychologischen Entspannungeffekt über die Umgebungsluft zu erzielen. D.h. anstelle der topischen Applikation kosmetischer Zubereitungen kann die Bereitstellung der Duftstoffe in der Umgebunsgluft auch in Form von Duftkerzen, Öllämpchen oder Raumbeduftungssystemen erfolgen.
Der Anteil an zusätzlichen Parfumbestandteilen in der Zubereitung beträgt vorteilhaft nicht mehr als 1 Gew.%, bezogen auf die Gesamtmasse der Zubereitung. Die zusätzlichen Parfümbestandteile sind Parfümrohstoffe, welche üblicherweise in Parfüms eingesetzt werden und z.B. in S. Arctander, "Perfume and Flavor Chemicals" (Montclair, N.J.,1969), S.Arctander, "Perfume and Flavor Materials of Natural Orgin" (Elizabeth, N.J.,1960) oder "Flavor and Fragrance Materials - 1991", Allured Publishing Co. Wheaton, USA beschrieben sind.
Bevorzugt ist es möglich, wenn erwünscht, 0,001 Gew.% bis maximal 0,1 Gew. % , bezogen auf die Gesamtmasse der Zubereitung, der folgenden Parfumsubstanzen der kosmetischen Zubereitung zusätzlich zu zusetzen:
Methyl dihydrojasmonate, Phenylethyl alcohol, Linalool, Linalyl acetate, 2,6-Dimethyl-7-octen-2-ol, isobornyl acetate, Isopropyl Myristate, Cashmeran, beta- ionone, decanal /aldehyde c-10, Acetone-Dihydroxytoluenbe Diether, Ethylvanilin, Ambrox, hexyl cinnamal, 2-Acetonapthone-1,2,3,4,5,6,7,8-octahydro-2,3,8,8-tetramethyl, Butylphenyl Methylpropional, Benzyl acetate, 1,3,4,6,7,8-Hexahydro-4,6,6,7,8,8-hexamethylcyclopenta-gamma-2-benzopyran, Methyl cedryl ketone, Ethylene brassylate, 4-(4-Hydroxy-4-methylpentyl)-3-cyclohexene-1-carboxyaldehyde, Benzyl salicylate, Hexyl salicylate, Orange oil, alpha-Isomethyl Ionone, 4-t-Butylcyclohexyl acetate, Patchouli oil, Geraniol, Tetrahydrolinalool, Hydroxycitronellal, Citronellol, Orange terpenes, Heliotropin, Terpinyl acetate, omega-Pentadecalactone, Methyl-alpha-ionone, Lavandin oil, Lemon oil, Bergamot oil, 7-Acetyl-1,1,3,4,4,6-hexamethyltetralin, Coumarin, Ethyllinalool, Amyl salicylate, 2-tert-Pentyl-cyclohexyl acetate, 3-Methyl-5-phenyl-1-pentanol, Cedrol, Benzyl benzoate, Vanillin, Amyl Cinnamal, Cedrol, Limonene, 2-Isobutyl-4-hydroxy-4-methyltetrahydropyran, Terpineol, Lavender oil, Benzyl Alcohol, Citral, Eugenol, Isoeugenol, Cinnamyl Alcohol, Farnesol, Cinnamal, Anise Alcohol, Evernia Prunastri (Extract),Benzyl Cinnamate, Methyl 2-Octynoate, Amylcinnamyl Alcohol,Evernia Furfuracea (Extract),Aldehyde C14,Hexenol cis 3,Decalactone Gamma,Helional,Hexenylacetate cis-3, Thymol, t-Anethole.

Bei Duftrohstoffen handelt es sich um Einzelriechstoffe, welche alle einen sehr speziellen Eigengeruch aufweisen. Generell ist daher erst durch die Kombination einer Vielzahl solcher Einzelriechstoffe ein komplexer Dufteindruck zu erzielen. Es ist demnach nicht verwunderlich, das Parfüms durchaus aus einer Vielzahl von Einzelriechstoffen bestehen, deren Anzahl auch bis über 100 gehen kann.
Mit den hier beschriebenen Duftstoffen ist durchaus ein parfümistischer Eindruck zu erzielen, welcher aber durch die Kombination mit den ggf. zusätzlichen Parfuminhaltsstoffe komplexer gestaltet werden kann, ohne die erfindungsgemäß beruhigende Wirkung zu schwächen.
Die hier genannten zusätzlichen typischen Parfuminhaltsstoffe können bei Bedarf eingesetzt werden, um den Duftcharakter des Parfums leicht abzurunden oder zu ergänzen und etwas voller zu machen.
Durch die Mengenbegrenzung der zusätzlichen Parfumbestandteile wird aber gewährleistet, dass der erfindungsgemäße Zweck der Beruhigungswirkung über die erfindungsgemäßen Stoffe nicht überdeckt oder sogar verhindert wird.

Als kosmetische Zubereitungen sind oftmals Emulsionen, hier insbesondere W/O-, O/W- oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert.
Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.
Die bevorzugte Applikationsform erfindungsgemäßer Stoffe erfolgt über topisch applizierbare Zubereitungen auf Basis einer Emulsion, bevorzugt einer Öl in Wasser Emulsion, einem Gel oder Hydrodispersion.

Die kosmetischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften hinsichtlich der Beruhigung und/oder Entspannung nicht beeinträchtigt.

Die folgenden Beispiele sollen die Erfindung näher beschreiben, ohne einschränkend zu wirken. Die Anteilsangaben beziehen sich auf Gewichtsanteile, bezogen auf die Gesamtmasse der Zubereitung, sofern nichts anderes offenbart ist.

**W/S-Emulsion**

| | **31** | **32** | **33** | **34** | **35** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| cyclisches Silikonöl (Cyclomethicon) | 12,5 | 15 | 22,0 | 20,0 | 15,5 |
| lineares Silikonöl (Dimethicon) | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Kaliumsorbat | 0,4 | 0,1 | 0,05 | 0,3 | 0,4 |
| Microcrystalline Cellulose | 1,0 | 0,1 | 0,5 | 0,25 | 0,1 |
| Silicon Elastomer Gel | 2,5 | 12,5 | 10 | 7,5 | 5,0 |
| Benzylalkohol | --- | --- | 0,05 | --- | 0,1 |
| modifizierte Stärke | --- | 2,5 | --- | 0,15 | -- |
| 2,6-Dimethyl-7-octen-2-ol | 0,03 | 2 | 0,01 | 0,5 | 1 |
| Ggf. weitere Parfum Rohstoffe | q,s, | q,s, | q,s, | q,s, | q,s, |
| Wasser | ad 100 | ad 100 | ad 100 | ad 100 | ad 100 |

## Patentansprüche

1. Kosmetische nichttherapeutische Verwendung des Stoffes 2,6-Dimethyl-7-octen-2-ol zur psychologischen Beruhigung und/oder Entspannung des erwachsenen Menschen **dadurch gekennzeichnet, dass** der Stoff in einer kosmetischen Zubereitung zur Applikation auf der Haut oder dem Haar eines Menschen angewendet wird.

2. Verwendung nach Anspruch 1 **dadurch gekennzeichnet, dass** die Zubereitung eine Emulsion, ein Gel oder eine Hydrodispersion ist.

3. Verwendung nach einem der vorstehenden Ansprüche, **dadurch gekennzeichnet, dass** der Stoff zu einem Anteil von mindestens 0,01 Gew.%, bezogen auf die Gesamtmasse der Zubereitung, eingesetzt wird.

4. Verwendung nach einem der vorstehenden Ansprüche, **dadurch gekennzeichnet, dass** der Anteil an zusätzlichen Parfumbestandteilen in der Zubereitung nicht mehr als 0,1 Gew.%, bezogen auf die Gesamtmasse der Zubereitung, beträgt.

## Claims

1. Cosmetic non-therapeutic use of the substance 2,6-dimethyl-7-octene-2-ol for psychological calming and/or relaxation of adult humans, **characterized in that** the substance is applied in a cosmetic preparation for application to the skin or hair of a human.

2. Use according to Claim 1, **characterized in that** the preparation is an emulsion, a gel or a hydrodispersion.

3. Use according to either of the preceding claims, **characterized in that** the substance is used at a proportion of at least 0.01% by weight, based on the total mass of the preparation.

4. Use according to any of the preceding claims, **characterized in that** the proportion of additional perfume constituents in the preparation is not more than 0.1% by weight, based on the total mass of the preparation.

## Revendications

1. Utilisation non thérapeutique cosmétique de la substance 2,6-diméthyl-7-octén-2-ol pour calmer et/ou détendre un être humain adulte au niveau psychologique, **caractérisée en ce que** la substance est utilisée dans une préparation cosmétique destinée à une application sur la peau ou les cheveux d'un être humain.

2. Utilisation selon la revendication 1, **caractérisée en ce que** la préparation est une émulsion, un gel ou une hydrodispersion.

3. Utilisation selon l'une quelconque des revendications précédentes, **caractérisée en ce que** la substance est utilisée en une proportion d'au moins 0,01 % en poids, par rapport à la masse totale de la préparation.

4. Utilisation selon l'une quelconque des revendications précédentes, **caractérisée en ce que** la proportion de constituants parfums supplémentaires dans la préparation n'est pas supérieure à 0,1 % en poids, par rapport à la masse totale de la préparation.
